# EUROPEAN PATENT APPLICATION

(11) **EP 0 650 714 A1**
(43) Date of publication of application: **03.05.1995**
(21) Application number: 93117656.4
(22) Date of filing: 01.11.1993
(51) Int. Cl.: A61F 13/15

(54) **Method of making an absorbent article using an activatable composite elastic member**

(71) Applicant: THE PROCTER & GAMBLE COMPANY, Cincinnati, Ohio 45202 (US)
(72) Inventor: Coles, Peter, D-65779 Kelkheim-Fischbach (DE); Divo, Michael, D-61381 Friedrichsdorf (DE); Soon, See-Aun, D-65824 Schwalbach (DE); Tamer, Attila, D-65824 Schwalbach am Taunus (DE)
(74) Representative: Bottema, Johan Jan

(57) **Abstract**

The invention relates to a method of making an absorbent article, in which a composite elastic member is attached to a topsheet, backsheet, core or any combination thereof. The composite elastic member comprises a first, relatively unextensible layer and a second, elastically extensible layer. The elastically extensible layer is attached to the unextensible layer in its relaxed state. The composite elastic member is supplied from a supply station while in its relatively unelongatable state and is activated by stretching or "ring rolling" just prior to, or shortly after attaching the composite elastic member to the topsheet, backsheet, core or any combination thereof. Alternatively, the method according to the invention comprises imparting extensibility to the composite elastic member in a manner such that the composite elastic member remains unelongatable in its longitudinal direction, followed by applying the composite elastic member to the topsheet, backsheet core or any combination thereof. The method according to the invention allows supply of an elastic member without need for tension control means and allows controlled design of the elastic properties of an absorbent article.

## Description

### FIELD OF THE INVENTION

The invention relates to method of making an absorbent article, the article having a liquid-pervious topsheet, a liquid-impervious backsheet and an absorbent core placed therebetween, the method comprising the step of applying an elastication means to the topsheet, the backsheet, the core or any combination thereof, the elastication means comprising a composite member having a first, relatively unextensible layer and a second, elastically extensible layer.

The invention also relates to an absorbent article comprising a composite elastic member and to a composite elastic member for use in a method of manufacturing an absorbent article.

### BACKGROUND OF THE INVENTION

From US-A-4 908 247, a shirrable composite elastic member is known which is comprised of a central, pre-stretched elastomeric layer such as a natural rubber layer, which is laminated to two outer layers. The outer layers consist for instance of extrusion-cast polystyrene and are relatively brittle but strong enough to maintain the central layer in its stretched condition. The elastic composite can be applied to a disposable absorbent article at low tension and high speed and is activated by mechanical manipulation so that elasticity is obtained in selective areas. The disposable absorbent article is, after activation of the elastic composite, gathered by the contraction of the composite elastic member. Although the above composite elastic member allows application to a disposable diaper in a continuous manufacturing process at relatively high speed, there are several aspects of the above composite elastic member that can be improved upon.

Firstly, the manufacture of the known composite elastic member requires the pretensioning of the central element, which complicates a continuous manufacturing process of the elastic composite as the central elastic element needs to be supplied in a pre-stretched state at a constant tension.

Secondly, for relatively weak or brittle outer layers, winding of the composite member onto a roll could lead to activation. Storage of the known composite member will therefore be relatively difficult.

Thirdly, the known composite elastic member will contract after activation by an amount which is in part determined by the percentage of elongation applied during pre-stretching of the central layer. The total amount of contraction can be controlled by activating a longer or shorter length of the composite elastic member. Contraction of the known composite member will cause the absorbent products, to which it is attached, to gather. For products in which no gathering or shirring is desired, the composite is less fit.

Finally, because of the contraction of the elastic member after activation, the absorbent product must be held under tension if activation occurs on line.

From US-A-5,167,897, a large number of composite elastic members is known comprising an elastically extensible layer laminated to a relatively unelongatable layer which is activated to become elongatable.

In US-A-3,025,199, an elastic net is disclosed comprising intersecting strands, two non-woven layers being connected to the net on either side thereof. Upon stretching of the elastic net, the non-woven layers are permanently elongated. When the elastic net returns to its relaxed state, the two non-woven layers are gathered and exhibit z-direction buckling.

In US-A-4,107,364 and US-A-4,209,563 a composite elastic member is described comprising an elastomeric non-woven and a relatively unextensible non-woven.

In US-A-4,525,407, a composite elastic member is disclosed comprising an elastic net laminated between two relatively unextensible films.

In US-A-5,167,897, it is described to attach an elastic member in its unextended state to a drawable topsheet and/or backsheet of a diaper and to permanently elongate the topsheet and the backsheet by passing these between intermeshing, corrugated rolls (ringrolling).

It is an object of the invention to provide a method of manufacturing an elasticated disposable absorbent article which allows application of a composite elastic member to an absorbent article, while the composite elastic member is in its substantially untensioned state.

It is a further object of the invention to provide a method of manufacturing an absorbent article in which the composite elastic member can be supplied at high speed from a storage roll, spool or storage box.

It is another object of the invention to provide a method of manufacturing an absorbent article in which the absorbent article can be maintained in its ungathered state after application and activation of the elastic composite member.

It is again another object of the invention to provide a method which allows production of an absorbent article at high speed and relatively low cost.

It is another object of the invention to provide a method of manufacturing an absorbent article by which stretchability can easily be provided in selected areas and in a selected direction, and in which the degree of extensibility can be easily controlled.

It is again an object of the invention to provide a stretchable elastic composite member which can be supplied in a constant, controlled manner, which can be efficiently stored and packaged and which allows for a high flexibility of design of the elastic characteristics of an absorbent article to which the elastic composite member is applied.

### SUMMARY OF THE INVENTION

The method according to the invention is characterised by:
- supplying the composite member from a supply station,
- transporting the composite member from the supply station to the topsheet, the backsheet, the core or any combination thereof in a direction of transport, the second, elastically extensible layer of the composite member being during transport attached to the first layer in a relatively unextended state, the composite member being during transport relatively unelongatable at least in the direction of transport,
followed by imparting a physical deformation to the composite member to render the composite member elastically elongatable.

The elastic composite member comprises a stretchable elastic film, scrim or other elastic element in its relatively relaxed state, to which elastic element a relatively unelongatable non-woven or other unelongatable layer is laminated with a substantially one-to-one lamination factor. The relatively unelongatable layer has an elastic modulus which is substantially larger than the elastic modulus of the elastic element, such that the total composite member is relatively unelongatable. The elastic composite member can be supplied from a roll or spool to the diaper in a high-speed manufacturing process in a similar way as a tape, topsheet or backsheet is supplied which does not have any elastically extensible properties. Hence there is no need for relatively complex tension-control means maintaining a constant percentage of stretch of the elastic composite member. Furthermore, variations in length which can occur upon transport of an elastic member, are reduced by the method according to the invention. Just shortly before attachment of the elastic composite member to the absorbent article, or after attachment thereto, the elastic composite member is activated. With "activated" it is meant that a physical deformation is applied to the unextensible layer, to impart a permanent elongation to this layer.

As the relatively unextensible layer and the elastically extensible layer are laminated with a substantially 1:1 ratio, i.e. no folds or gathers are present in either layer, the composite elastic member can be firmly gripped by vacuum gripper means, such as for instance a cut-and-slip applicator, which is commonly used in high-speed manufacturing of absorbent articles.

Another advantage of the 1:1 factor of lamination of the relatively unextensible layer to the elastic layer when the elastic layer is in its relaxed state, is that the composite elastic member according to the invention can be stored on a roll or festooned in a box, in a space-effective manner and that no air is included such as it is the case for a gathered elastic composite structure.

The composite elastic member can be activated by extending the member beyond its un-activated length, or by imparting a physical deformation pattern to the composite member such as a number of pleats or perforations.

When extensibility is provided to the composite elastic member by contacting the composite member with two intermeshing corrugated rolls, the first, relatively inelastic, layer absorbs a larger part of the energy imparted to the composite elastic member, such that the elastically extensible layer of the composite elastic member remains substantially undamaged.

By first selectively activating parts of the elastic composite member according to the invention, followed by pre-tensioning the composite elastic member and then attaching it to the topsheet, backsheet or core of an absorbent article, the absorbent article is gathered in predetermined areas corresponding to the activated parts of the composite elastic member.

Alternatively, the absorbent article can be gathered before attachment of the composite elastic member. In this case, the composite elastic member can be activated by ring rolling or stretching prior to attachment to the absorbent article, and can be attached thereto in its relaxed state. The composite elastic member can also be applied to the gathered absorbent article in its non-activated state, and be subsequently activated by extending the absorbent article to assume its flattened state. The areas in which elastication is imparted to the absorbent article can be determined by the bonding pattern with which the composite elastic member is attached to the absorbent article.

The composite elastic member can also be attached to a flattened, non-gathered absorbent article while the composite member is in its relaxed state, where after both the composite elastic member and the absorbent article are rendered extensible by contacting both composite elastic member and absorbent article with corrugated members, either in the shape of flat plates or rolls.

By attaching the composite elastic member to the absorbent article in its non-activated state and subsequently activating the composite elastic member, the amount of stretch will be determined by the nature and spatial configuration of the physical deformations that are applied. Contrary to known elastication methods in which the amount of stretch of the absorbent article is limited to the length by which the elastic member is pre-stretched, the amount of stretch of the composite elastic member according to the invention can be increased by as far as the total extensibility of the elastic layer in the composite member allows without breaking up to the point in which delamination of the layers of the composite elastic member occurs.

A further method according to the invention is characterized by:
- supplying the composite member from a supply station,
- transporting the composite member from the supply station to the topsheet, the backsheet, the core or any combination thereof in a direction of transport, the composite member being during transport relatively unelongatable in the direction of transport, followed by
- applying the composite member to the topsheet, the backsheet, the core or any combination thereof, wherein a physical deformation has been imparted to the composite member prior to applying it to the topsheet, the backsheet the core or any combination thereof to render at least one area of the composite member elastically elongatable such that the composite elastic member remains relatively unelongatable in the direction of transport.

The composite elastic member can for instance be provided with corrugations which extend in the direction of the length of the composite elastic member. Hereby, the composite elastic member is elastically extensible in a direction which is perpendicular to the direction of transport, while the composite elastic member is relatively unextensible in the direction of transport. Alternatively, the composite elastic member is along its length activated in areas such that a central, longtitudinal band of un-activated material remains to prevent longitudinal extensibility.

The composite elastic member can be directly attached to the topsheet, backsheet or core without imparting futher physical deformations or can be provided with additional deformations to render the member elastically elongatable in the direction of transport.

The composite elastic member according to the invention can comprise a number of components of an absorbent article. The term absorbent article covers disposable articles which are intended to be discarded after use and which comprise baby diapers, adult incontinence products and training pants, sanitary napkins, panty liners and light incontinence products. Baby diapers, adult incontinence products and training parts will hereafter be referred to as 'diapers'. Panty liners and light incontinence products will be referred to as 'sanitary napkins'. The composite elastic member according to the invention can comprise:
- Leg or waist elastics in a diaper, adult incontinence products or training parts, for forming an elastic seal around the legs or waist of a wearer. Leg elastics have been described in US-A-3,860 003 and US-A-4,081,301.
- Stand-up barrier cuffs comprising elastic spacing means for spacing away from the topsheet a free edge of the cuffs. The stand-up barrier cuffs can be located along the longitudinal edges of a diaper (barrier leg cuffs), along the transverse edges (barrier waist cuffs) or along both edges to form a containment pocket. Stand-up barrier cuffs have been described in detail in US-A- 5,087,255, US-A-4,938,755 and US-A-4,795,454.
- Elasticated side panels of a diaper. The elastication means can comprise separately attached ears or can be comprised between the topsheet and the backsheet in the region of the side panels. Elasticated side panels are described in US-A-5,156,793.
- The topsheet or backsheet or a part thereof of a diaper or a sanitary napkin. The topsheet or backsheet comprising or consisting of a composite elastic member according to the invention, can be provided with regions of different extensibility by selectively activating parts of the topsheet and/or backsheet while leaving other parts un-activated and hence unelongatable.
- Longitudinal shaping elastics on a sanitary napkin for importing a cup-like shape thereto. Elasticated sanitary napkins have been described in US-A-5,234,422.
- Elastically extensible wings of a sanitary napkin. Preferably, the composite elastic material is formed of a strip of material which is attached to the backsheet of a sanitary napkin and extends beyond the longitudinal sides of the napkin to form a pair of wings.

### BRIEF DESCRIPTION OF THE DRAWINGS

The invention will be described in detail with reference to the accompanying drawings. In the drawings:
Figure 1 shows a schematic cross-sectional view of a composite elastic member before activation,
Figures 2 and 3 show a schematic cross-sectional view of a composite elastic member after activation,
Figure 4 shows a preferred embodiment of a composite elastic member according to the invention,
Figures 5-10 show embodiments of a method for making an absorbent article comprising the step of attaching a composite elastic member according to the invention,
Figures 11 and 12 show a partial plan view of a composite elastic member according to the invention,
Figure 13 shows a partially cut-away plan view of an absorbent article,
Figure 14 shows a cross-sectional view of the absorbent article of Figure 13 along the line 2-2,
Figure 15 shows a plan view of a disposable diaper comprising a composite elastic member in side panel regions, waist regions and leg regions,
Figure 16 shows a perspective view of a training pants having a composite elastic member in side panel regions, waist regions, leg regions and in the crotch region,
Figure 17 shows a plan view of a sanitary napkin having wings comprising a composite elastic member,
Figure 18 shows a cross-sectional view of the sanitary napkin of figure 17 along the line 70-70,
Figure 19 shows a plan view of a sanitary napkin comprising along its longitudinal edges a composite elastic member,
Figure 20 shows a cross-sectional view of the sanitary napkin of Figure 15 along the line 83-83 and
Figure 21 shows a plan view of a sanitary napkin comprising a composite elastic member which is provided with areas of extensibility.

### DETAILED DESCRIPTION OF THE INVENTION

As used herein, the term "absorbent article" refers to devices which absorb and contain body exudates, and, more specifically, refers to devices which are placed against or in proximity to the body of the wearer to absorb and contain the various exudates discharged from the body. The term "disposable" is used herein to describe absorbent articles which are not intended to be laundered or otherwise restored or reused as an absorbent article (i.e., they are intended to be discarded after a single use and, preferably, to be recycled, composted or otherwise disposed of in an environmentally compatible manner). A "unitary" absorbent article refers to absorbent articles which are formed of separate parts united together to form a coordinated entity so that they do not require separate manipulative parts like a separate holder and liner. The present invention is also applicable to other absorbent articles such as incontinent briefs, incontinent undergarments, diaper holders and liners, feminine hygiene garments, and the like.

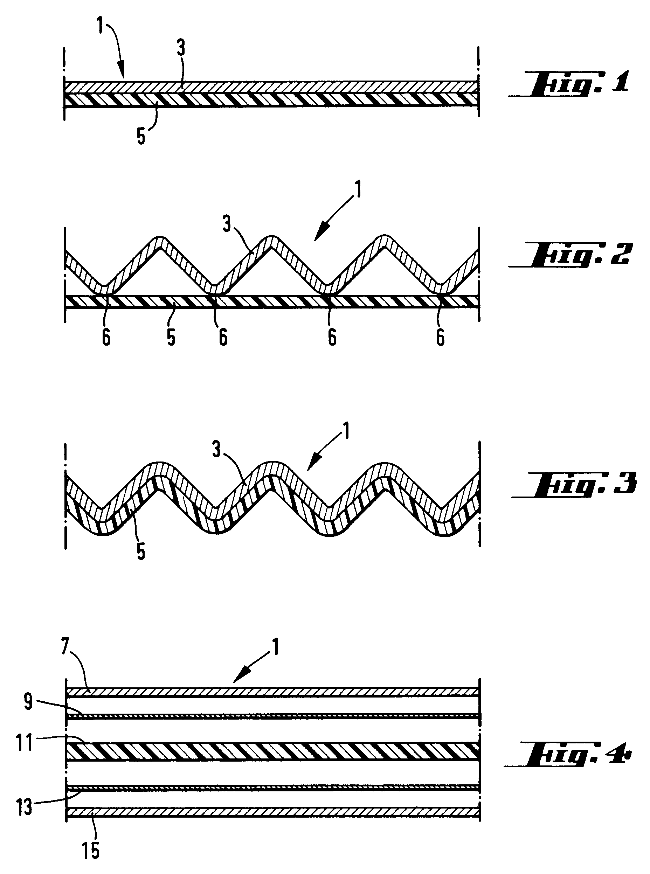

Figure 1 shows a composite elastic member 1 comprising a first layer 3 which is a relatively unextensible or unelongatable non-woven layer or a film. With relatively unextensible or unelongatable it is meant that the layer 3 has an elongation of not more than 5 % at a force between 0.5 N/cm and 5 N/cm, preferably between 1.5 N/cm and 3 N/cm. The layer 3 can be comprised of a non-woven, a thermoplastic film, paper or cardboard or a combination thereof.

The layer 3 is bonded to an elastically extensible layer 5. The layer 5 can be an elastomeric film, an elastomeric non-woven layer or an elastomeric adhesive and has an elongation of about 40 % in a first load cycle when subjected to a force of between 0.6 N/cm and 1.2 N/cm, and an elongation of about 40% in a second unload cycle when subjected to a force between 0.3 N/cm and 0.8 N/cm.

The layers 3 and 5 can be bonded along their contiguous surfaces by adhesive, ultrasonic or fusion bonds. The bond areas in which the layers 3 and 5 are connected can comprise discrete areas or can cover the whole of the contiguous surfaces of layers 3 and 5. Elasticity is imparted to the composite elastic member 1 by permanently elongating or rupturing the layer 3, which process is hereafter referred to as 'activation'.

Before activating the layers 3 and 5, the composite elastic member 1 is relatively unextensible and has at least approximately the same modulus of elasticity as the modulus of the non-elastic layer 3. The modulus of elasticity of the composite elastic member 1 is adapted so that the elastic member 1 can be supplied from a supply station, in a process for manufacturing a disposable absorbent article, in a non-elongatable state at a tension of about 0.05 N/cm or more.

Before activation, the surface areas of the non-elastic layer 3 and the elastically extensible layer 5 are equal, both layers being in a relatively flattened state. The strength of the bond between the layers 3 and 5 after activation should be higher than 1 N /cm, which is strong enough to prevent separation of the layers in the process of making an absorbent article or upon use of the absorbent article. The above minimum bond strength is measured after pre-stretching the non-activated composite elastic member by 200 %, followed by relaxation of the composite elastic member.

Activation of the composite elastic member 1 can occur by imparting a physical deformation to the inelastic layer 3, which renders the layer 3 permanently elongatable. Activation can be imparted by stretching the layer 3. In case layer 3 is formed by a non-woven, stretching the non-woven will cause the fibers in the layer 3 to become disentangled to a certain extent or will cause a number of fibers to break. In case layer 3 is a film, stretching the film 3 will impart plastic deformation and cause the film to permanently elongate. The layer 3 can be provided with lines of weakening extending across the width of the layer, such as perforations or score-lines, along which the layer 3 will rupture upon applying a tensile force of sufficient magnitude. The activation force at which elongation of the layer 3 occurs is at least 5 N/cm.

Alternatively, the unextensible layer 3 can be provided with a two-dimensional pattern of perforations, by which the layer is sufficiently weakened to become elongatable.

Preferably, the composite elastic member 1 is activated by passing the layers 3 and 5 between two corrugated rolls having intermeshing grooves and ridges. This causes the layer 3 to become elongatable in a harmonica-like fashion. The direction of extensibility of the layer will be in a direction, perpendicular to the ridges of the corrugated rolls. The extent of the extensibilty will be determined by the interspacing of the ridges of the corrugated rolls. By adapting the pattern of the corrugations of the rolls, the direction and the degree of extensibilty of the composite elastic member can be modified. For instance, in the waist area of a disposable diaper, more extensibility and lower elastic forces are required than for instance at the side panel areas of the diaper. A process for imparting physical deformations, generally referred to as ring rolling, has been described in detail in US-A-5,143,679, US-A-5,167,897 and WO 92/15445.

As shown in figure 2, activation of the layer 3 causes the layer to be gathered between bond areas 6. Upon activation, the layer 3 is partly detached from the layer 5 but remains attached in bond areas 6. Alternatively, the layer 3 is before activation only attached in bond areas 6, and remains attached thereto after activation. The ratio of surface-areas of the upper layer 3 and lower layer 5 after activation can be between 1.2 and 4, corresponding to an extensibility of between 20 and 400 %.

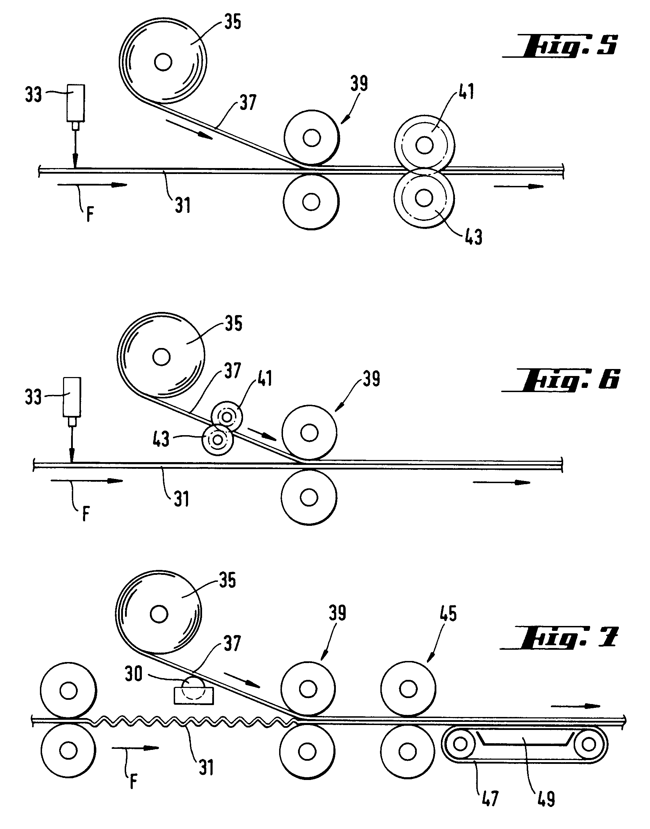

In case the elastic layer 5 is elongated upon activation, the upper layer 3 can remain attached to the layer 5 with its complete surface area. When the elastic layer 5 is formed by a thin layer of elastomeric adhesive, the layer 5 can follow the deformations of the upper layer 3. The ratio of the surface areas of inelastic layer 3 and the elastic layer 5 will in this case remain unaltered after activation. This is shown in Figure 3.

In figure 4 a preferred embodiment of a composite elastic member 1 is shown in which two unextensible layers 7,15 are bonded to a central elastic layer 11 by means of layers of adhesive 9,13. Layers 7 and 15 are comprised of polyethylene non-woven of denier 1.8 and basis weight of 20 g/m2.

The adhesive layers 9,13 are for instance comprised of Styrene-Isoprene-Styrene (SIS) or Styrene-Butadiene-Styrene (SBS) types of pressure-sensitive adhesive, such as manufactured by Findley Euro B.V., Rotkreuzweg 7, D-6380 Bad Homburg, Germany under type number H2275. The adhesive is applied by a meltblown process, spiral glue application, slot coating or other processes.

The central layer 11 is an elastomeric film such as available from Exxon Chemical Company, 351 N Oakwood Road, Lake Zurich, Illinois 60047-1562, under type number Exxon 500D.

The elastic composite 1 has a caliper of about 0.6 mm at a pressure of 8 g/cm2.

Alternatively, the non-elastic layers 7 and 15 are connected to the central elastic layer 11, by directly bonding layer 7 to layer 15 through perforations in the layer 11. This can be achieved by compressing the layers 7 and 9 in discrete points in a nip between patterned rolls and perforating the central layer 11 in these points, for instance ultrasonically. Upon application of heat and pressure to the layers 7 and 15 in these discrete points, the layer 11 will melt to a sufficient degree that layers 7 and 15 can connect through the molten elastomeric layer 11. A process of laminating two layers through a central elastically extensible layer has been described in WO 91/04724.

Figure 5 shows a web 31 which travels in the direction of the arrow F. The web 31 can consist of a topsheet, a backsheet, a fluff pulp core or a combination thereof. To the web 31, a layer of adhesive is applied by means of a glue applicator 33, which can be a nozzle for depositing a spiral pattern of glue lines. From a supply roll 35, the composite elastic member 37 is transported to the web 31 while being maintained in its inn-activated state such that it is relatively unextensible. The tension applied to the elastic member 37 is about 0.1 N/cm2 or higher and is kept low enough not to exceed the activation tension of the composite elastic member.

After the elastic member 37 is attached to the web 31, the elastic member and the web are combinedly passed between two intermeshing rolls 41, 43. The rolls 41, 43 comprise a number of parallel circumferential grooves and rims which deform the web 31 and the elastic member 37 along straight or curved lines in the longitudinal or transverse direction of the web. The grooves and rims may extend perpendicular to the plane of drawing to impart longitudinal extensibility, or may extend parallel to the plane of the drawing to impart extensibility to the web 31 and the composite elstic member in a direction which is perpendicular to the plane of the drawing. When the web 31 is extended in this direction, the elastic layer of the composite elastic member 37 provides a restoring force.

In the laminating nip 39, the elastic member 37 is subjected to temperatures of for instance 135°C and, optionally, high lamination pressures. The unextensible layer of the composite elastic member 37 absorbs a large part of the energy imparted to the elastic member 37 during attachment to the web 31, so that the elastic layer of the composite elastic member is not damaged upon lamination. Also between the intermeshing rolls 41, 43, the unextensible layer absorbs a large part of the activation energy, such that damage to the elastic layer is prevented.

In the embodiment of Fig. 6, the elastic member 37 is activated just prior to joining it to the web 31 in the nip 39. The activated elastic member can be attached in its unextended state, in which case the web 31 is foreshortened or gathered, to provide extensibility. Alternatively, the activated member is applied in prestretched manner, for instance by rotating the rolls 41, 43 at such a speed that the velocity of the elastic member towards the rolls 41, 43 is lower than the velocity of the elastic member away from the rolls.

In the embodiment of Fig. 7, the web 31 is gathered prior to attaching the composite elastic member 37. Adhesive is applied to the elastic member 37 by means of a gravure printing roll 30. Activation of the elastic member 37 occurs between the laminating nip 39 and the stretching nip 45, the feed velocity of which is higher than the feed velocity through the laminating nip 39. The web 31 and the elastic member 37 are transported in their extended state by means of a perforated conveyor 47 running over a suction box 49.

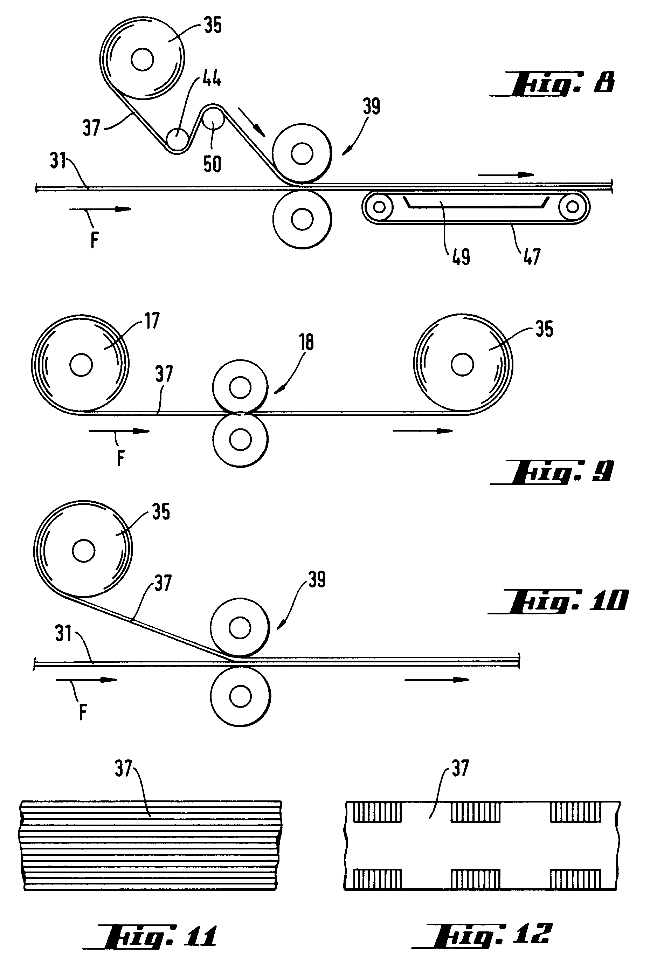

In the embodiment of Fig. 8, the elastic member 37 is activated before attaching it to the web 31, by pre-stretching it between two rollers 40, 42 and is subsequently attached to the web 31 in its pre-stretched state.

Figure 9 shows a method in which the composite elastic member 37 is provided with a physical deformation by passing the member 37 between the pair of patterned rolls 18. The composite elastic member 37 is unwound from a first roll 17, and is fed into the nip of rolls 18. The composite elastic member 37 is along its length provided with a pattern of corrugations or pleats as shown in figures 11 and 12, and is subsequently wound onto a supply roll 35. The elastic member 37 can be stored or transported on the supply roll 35. The physical deformations of the type as shown in figures 11 and 12, do not impart significant extensibility to the elastic member 37 in its longitudinal direction. Hence the elastic member 37 can be unwound from the supply roll 35, transported to the web 31 and laminated thereto, while being non-extensible in the direction of transport of the composite elastic member 37, as is shown in figure 10.

Generally, the composite elastic member 37 can before attachment to the web 31 be provided with physical deformations which provide extensibility to the composite elastic member in the longitudinal direction of the member 37, provided that these physical deformations do not extend across the entire widh of the composite elastic member, as shown in figure 11. Alternatively, the composite elastic member 37 can before attachment to the web 31 be provided with extensibility in a direction which is transverse to length of the composite elastic member, as shown in figure 10.

Figure 13 is a plan view of the diaper 20 of the present invention in its flat-out, state (i.e., with elastic induced contraction pulled out) with portions of the structure being cut-away to more clearly show the construction of the diaper 20 and with the portion of the diaper 20 which faces or contacts the wearer, the inner surface, oriented towards the viewer. As shown in Figure 13, the diaper 20 preferably comprises a liquid-pervious topsheet 24; a liquid-impervious backsheet 26 joined with the topsheet 24; an absorbent core 28 positioned between the topsheet 24 and the backsheet 26; side panels 29; elasticized leg cuffs 32; an elastic waist feature 34 and a fastening system generally multiply designated as 36. The inner surface of the diaper 20 comprises that portion of the diaper 20 which is positioned adjacent to the wearer's body during use (i.e., the inner surface generally is formed by at least a portion of the topsheet 24 and other components joined to the topsheet 24). The outer surface comprises that portion of the diaper 20 which is positioned away from the wearer's body (i.e., the outer surface generally is formed by at least a portion of the backsheet 26 and other components joined to the backsheet 26).

Figure 13 shows a preferred embodiment of the diaper 20 in which the topsheet 24 and the backsheet 26 have length and width dimensions generally larger than those of the absorbent core 28. The topsheet 24 and the backsheet 26 extend beyond the edges of the absorbent core 28 to thereby form the periphery 30 of the diaper 20. While the topsheet 24, the backsheet 26, and the absorbent core 28 may be assembled in a variety of well known configurations, preferred diaper configurations are described generally in U.S. Patent 3,860,003 entitled "Contractable Side Portions for Disposable Diaper" which issued to Kenneth B. Buell on January 14, 1975; and U.S. Patent Application Serial No. 07/715,152, allowed, "Absorbent Article With Dynamic Elastic Waist Feature Having A Predisposed Resilient Flexural Hinge", Kenneth B. Buell et al. filed June 13, 1991; each of which is incorporated herein by reference.

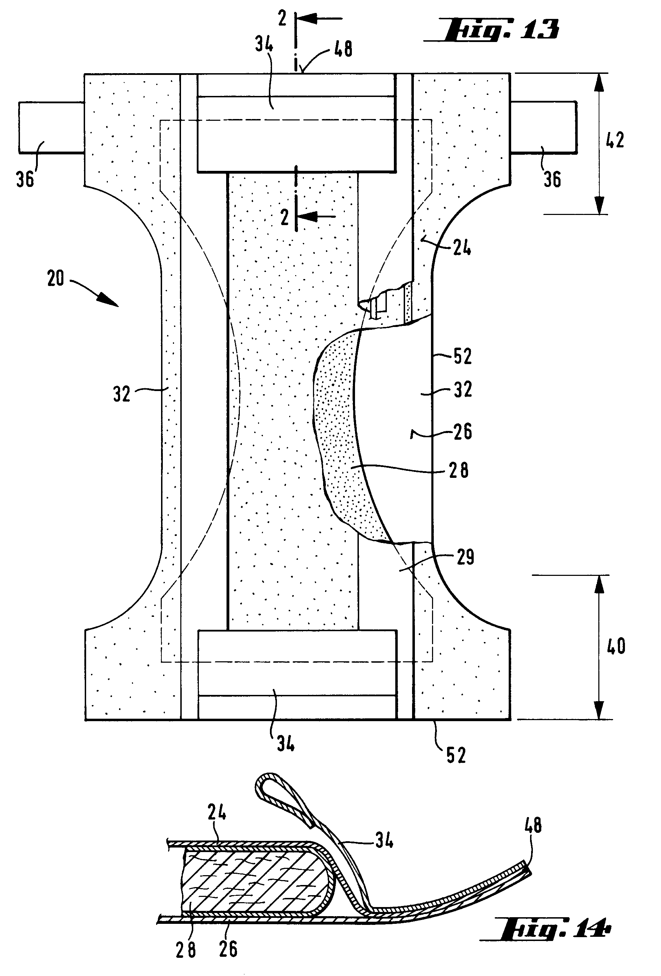

Figure 14 is a cross-sectional view of the diaper 20 taken along section line 2-2 of Figure 13.

The absorbent core 28 may be any absorbent means which is generally compressible, conformable, non-irritating to the wearer's skin, and capable of absorbing and retaining liquids such as urine and other certain body exudates. As shown in figure 13, the absorbent core 28 has a garment surface, a body surface, side edges, and waist edges. The absorbent core 28 may be manufactured in a wide variety of sizes and shapes (e.g., rectangular, hourglass, "T"-shaped, asymmetric, etc.) and from a wide variety of liquid-absorbent materials commonly used in disposable diapers and other absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; tissue including tissue wraps and tissue laminates; absorbent foams; absorbent sponges; superabsorbent polymers; absorbent gelling materials; or any equivalent material or combinations of materials. The configuration and construction of the absorbent core may also be varied (e.g., the absorbent core may have varying caliper zones, a hydrophilic gradient, a superabsorbent gradient, or lower average density and lower average basis weight acquisition zones; or may comprise one or more layers or structures). The total absorbent capacity of the absorbent core 28 should, however, be compatible with the design loading and the intended use of the diaper 20. Further, the size and absorbent capacity of the absorbent core 28 may be varied to accommodate wearers ranging from infants through adults. Exemplary absorbent structures for use as the absorbent core 28 are described in U.S. Patent 4,610,678 entitled "High-Density Absorbent Structures" issued to et al. on September 9, 1986; U.S. Patent 4,673,402 entitled "Absorbent Articles With Dual-Layered Cores" issued to Weisman et al. on June 16, 1987; U.S. Patent 4,888,231 entitled "Absorbent Core Having A Dusting Layer" issued to Weisman on December 19, 1989; and U.S. Patent 4,834,735, entitled "High Density Absorbent Members Having Lower Density and Lower Basis Weight Acquisition Zones", issued to Alemany et al. on May 30, 1989. Each of these patents are incorporated herein by reference.

The backsheet 26 is positioned adjacent the garment surface of the absorbent core 28 and is preferably joined thereto by attachment means (not shown) such as those well known in the art. For example, the backsheet 26 may be secured to the absorbent core 28 by a uniform continuous layer of adhesive, a patterned layer of adhesive, or an array of separate lines, spirals, or spots of adhesive. Adhesives which have been found to be satisfactory are manufactured by H. B. Fuller GmbH, An der Roten Bleiche 2-3, D-2120 Luneburg, Germany and marketed as HL-1258. The attachment means will preferably comprise an open pattern network of filaments of adhesive as is disclosed in U.S. Patent 4,573,986 entitled "Disposable Waste-Containment Garment", which issued to Minetola et al. on March 4, 1986 more preferably several lines of adhesive filaments swirled into a spiral pattern such as is illustrated by the apparatus and methods shown in U.S. Patent 3,911,173 issued to Sprague, Jr. On October 7, 1975; U.S. Patent 4,785,996 issued to Ziecker, et Al. on November 22, 1978; and U.S. Patent 4,842,666 issued to Werenicz on June 27, 1989. Each of these Patents are incorporated herein by reference. Alternatively, the attachment means may comprise heat bonds, pressure bonds, ultrasonic bonds, dynamic mechanical bonds, or any other suitable attachment means or combinations of these attachment means as are known in the art.

The backsheet 26 is impervious to liquids (e.g., urine) and is preferably manufactured from a thin plastic film, although other flexible liquid impervious materials may also be used. As used herein, the term "flexible" refers to materials which are compliant and will readily conform to the general shape and contours of the human body. The backsheet 26 prevents the exudates absorbed and contained in the absorbent core 28 from wetting articles which contact the diaper 20 such as and undergarments. The backsheet 26 may thus comprise a woven or material, polymeric films such as thermoplastic films of polyethene or polypropylene or, or composite materials such as a film-coated nonwoven material. Preferably, the backsheet is a film having a thickness of from about 0.012mm (0.5mil) to about 0.051mm (2.0mils). Particularly preferred materials for the backsheet include RR8220 blown films and RR5475 cast films as manufactured by Tredegar Industries, Inc. of Terre Haute, IN. The backsheet 26 is preferably embossed and/or matte finished to provide a more clothlike appearance. Further, the backsheet 26 may permit vapors to escape from the absorbent core 28 (i.e.breathable,) while still preventing exudates from passing through the backsheet 26.

The topsheet 24 is positioned adjacent the body surface of the absorbent core 28 and is preferably joined thereto and to the backsheet 26 by attachment means (not shown) such as those well known in the art. Suitable attachment means are described with respect to joining the backsheet 26 to the absorbent core 28. As used herein, the term "joined" encompasses configurations whereby an element is directly secured to the other element by affixing the element directly to the other element, and configurations whereby the element is indirectly secured to the other element by affixing the element to intermediate member(s) which in turn are affixed to the other element. In a preferred embodiment of the present invention, the topsheet 24 and the backsheet 26 are joined directly to each other in the diaper periphery 60 and are indirectly joined together by directly joining them to the absorbent core 28 by the attachment means (not shown).

The topsheet 24 is compliant, soft feeling, and non-irritating to the wearer's skin. Further, the topsheet 24 is liquid pervious permitting liquids (e.g., urine) to readily penetrate through its thickness. A suitable topsheet may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene or fibers), or a combination of natural and synthetic fibers. Preferably, the topsheet 24 is made of a hydrophobic material to isolate the wearer's skin from liquids contained in the absorbent core 28. There are a number of manufacturing techniques which may be used to manufacture the topsheet 24. For example, the topsheet 24 may be a nonwoven web of fibers, spunbonded, carded, wet-laid, meltblown, hydroentagled, combinations of the above, or the like. A preferred topsheet is carded and thermally bonded by means well known to those skilled in the fabrics art. A preferred topsheet comprises a web of staple length polypropylene fibers such as is manufactured by Veratec, Inc., a Division of International Paper Company, of Walpole, Massachusetts under the designation P-8.

The diaper 20 preferably further comprises elasticized leg cuffs 32 for providing improved containment of liquids and other body exudates. Each elasticized leg cuff 32 may comprise several different embodiments for reducing the leakage of body exudates in the leg regions. (The leg cuff can be and is sometimes also referred to as leg bands, side flaps, barrier cuffs, or elastic cuffs.) U.S. Patent 3,860,003 describes a disposable diaper which provides a contractible leg opening having a side flap and one or more elastic members to provide an elasticized leg cuff (gasketing cuff). U.S. Patent 4,909,803 entitled "Disposable Absorbent Article Having Elasticized Flaps" issued to Aziz et al. on March 20, 1990, describes a disposable diaper having "stand-up" elasticized flaps (barrier cuffs) to improve the containment of the leg regions. U.S. Patent 4,695,278 entitled "Absorbent Article Having Dual Cuffs" issued to Lawson on September 22, 1987, describes a disposable diaper having dual cuffs including a gasketing cuff and a barrier cuff.

The Diaper 20 preferably further comprises an elastic waist feature 34 that provides improved fit and containment. The elastic waist feature 34 is that portion or zone of the diaper 20 which is intended to expand and contract to dynamically fit the wearer's waist. The elastic waist feature 34 at least extends longitudinally outwardly from at least one of the waist edges of the absorbent core 28 and generally forms at least a portion of the end edge 48 of the diaper 20. Disposable diapers are generally constructed so as to have two elastic waist features, one positioned in the first waist region 42 and one positioned in the second waist region 40, although diapers can be constructed with a single elastic waist feature. Further, while the elastic waist feature or any of its constituent elements can comprise a separate element affixed to the diaper 20, the elastic waist feature 34 is preferably constructed as an extension of other elements of the diaper such as the backsheet 26 or the topsheet 24, preferably both the backsheet 26 and the topsheet 24. The elasticized waist feature 34 may be constructed in a number of different configurations including those described in U.S. Patent 4,515,595 issued to Kievit et al. on May 7, 1985 and the above referenced U.S. Patent Application Serial No 07/715,152; each of these references being incorporated herein by reference.

The diaper 20 also comprises a fastening system 36 which forms a side closure which maintains a first waist region 42 and a second waist region 40 in an overlapping configuration such that lateral tensions are maintained around the circumference of the diaper to maintain the diaper on the wearer. Exemplary fastening systems are disclosed in U.S. Patent 4,846,815 entitled "Disposable Diaper Having An Improved Fastening Device" issued to Scripps on July 11, 1989; U.S. Patent 4,894,060 entitled "Disposable Diaper With Improved Hook Fastener Portion" issued to Nestegard on January 16, 1990; U.S. Patent 4,946,527 entitled "Pressure-Sensitive Adhesive Fastener and Method of Making Same" issued to Battrell on August 7, 1990; U.S. Patent 3,848,594 entitled "Tape Fastening System for Disposable Diaper" issued to Buell on November 19, 1974; U.S. Patent B1 4,662,875 entitled "Absorbent Article" issued to Hirotsu et al. on May 5, 1987; and the hereinbefore referenced U.S. Patent Application 07/715,152; each of which is incorporated herein by reference.

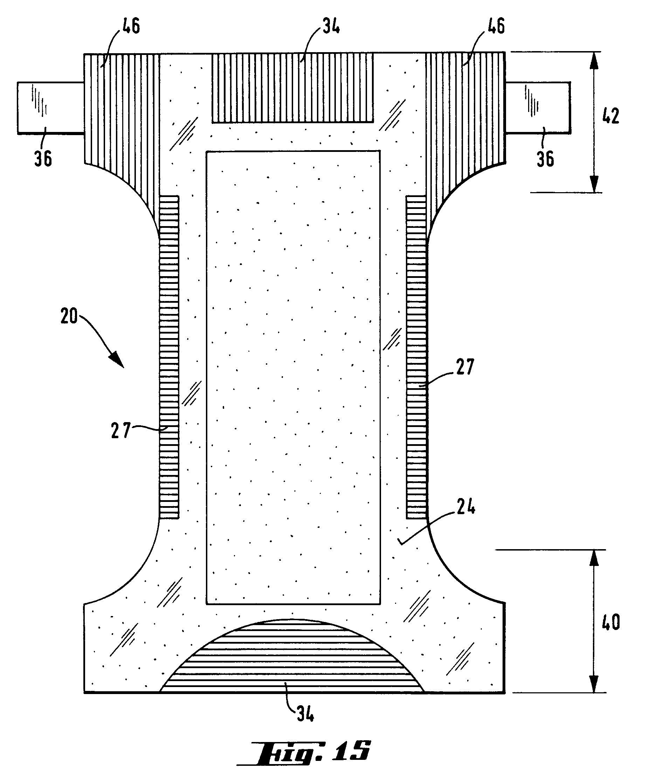

In a preferred embodiment, the diaper also comprises elasticized side panels 46 disposed in the first waist region 42, as shown in Figure 15. The elasticized side panels 46 provide an elastically extensible feature that provides a more comfortable and contouring fit by initially conformably fitting the diaper to the wearer and sustaining this fit throughout the time of wear well past when the diaper has been loaded with exudates since the elasticized side panels allow the sides of the diaper to expand and contract. The elasticized side panels 46 further provide more effective application of the diaper 20 since even if the diaper pulls one elasticized side panels farther than the other during application (asymmetrically), the diaper 20 will "self-adjust" during wear. The diaper of the present invention preferably has the elasticized side panels 46 disposed in the first waist region 42; alternatively, the diaper 20 may be provided with elasticized side panels 46 disposed in the second waist region 40 or in both the first waist region 42 and the second waist region 40.

While the elasticized side panels 46 may be constructed in a number of configurations, examples of diapers with elasticized side panels positioned in the ears (ear flaps) of the diaper are disclosed in U.S. Patent 4,857,067, entitled "Disposable Diaper Having Shirred Ears" issued to Wood, et al. on August 15, 1989; U.S. Patent 4,381781 issued to Sciaraffa, et al. on May 3, 1983; U.S. Patent 4,938,753 issued to Van Gompel, et al. on July 3, 1990; and the hereinbefore referenced U.S. Patent Application Serial No. 07/715,152; each of which are incorporated herein by reference.

Figure 15 shows a disposable diaper 20 to which the composite elastic member 37 is attached in the side panel regions 46, the waist feature regions 34 and /or the areas 27 defining the leg openings upon use.

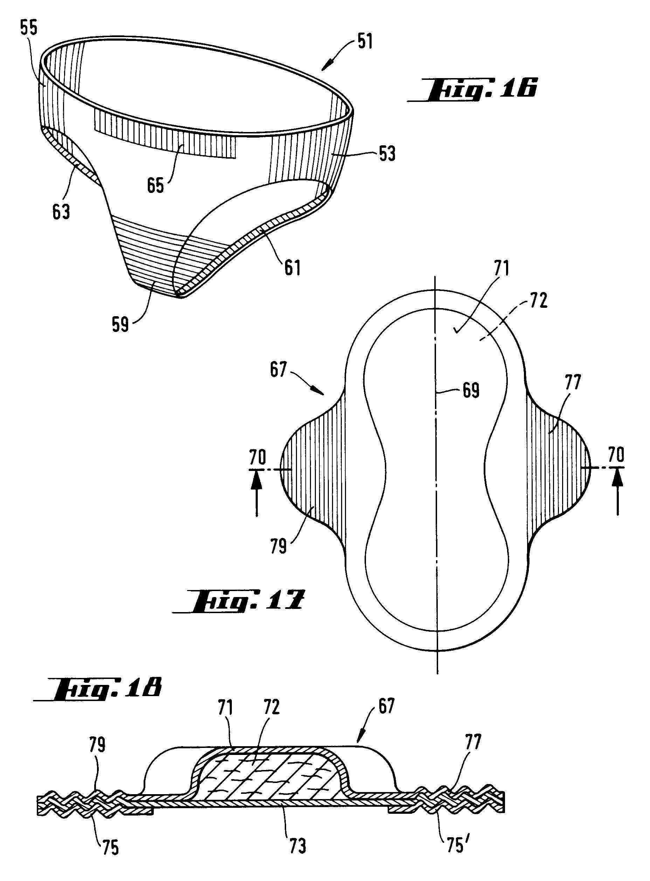

Fig. 16 shows a training pants, which comprises the composite elastic member 37 in side panels 53, 55, around the margins of the leg openings 61, 63, in the crotch area 59 and in the waist region 65. The amount of extensibility and the elasticity in the different elasticated areas of training pants 50 can easily be adjusted by varying the number and/or spacing of the parallel activation lines, or by adjusting the depth of the intermeshing rims and grooves of the rolls 41, 43.

Fig. 17 shows a sanitary napkin 67 which has an hour-glass shaped absorbent core 72. The topsheet 71 and the backsheet 73 extend beyond the core 72 to form wings 77, 79 for attachment of the sanitary napkin 67 to an undergarment. In the area of the wings 77, 79, the topsheet 71, the backsheet 73 and elastic member 75 are provided with parallel corrugations extending in the direction of the longitudinal center line 69.

As it can be seen in Fig. 18, the composite elastic member 75 is attached to the backsheet 73 only in the areas of the wings 77, 79. Alternatively, the composite elastic member 75 is formed of a single piece of material extending along the whole of the width of the backsheet 73. It is also possible to use as a backsheet 73 a liquid-impermeable elastic member, so that no extra layer of material is required. In this case, the backsheet can be elasticated in specific areas, such at the wings 77, 79 or other areas located between the wings, by activation of the backsheet in these areas.

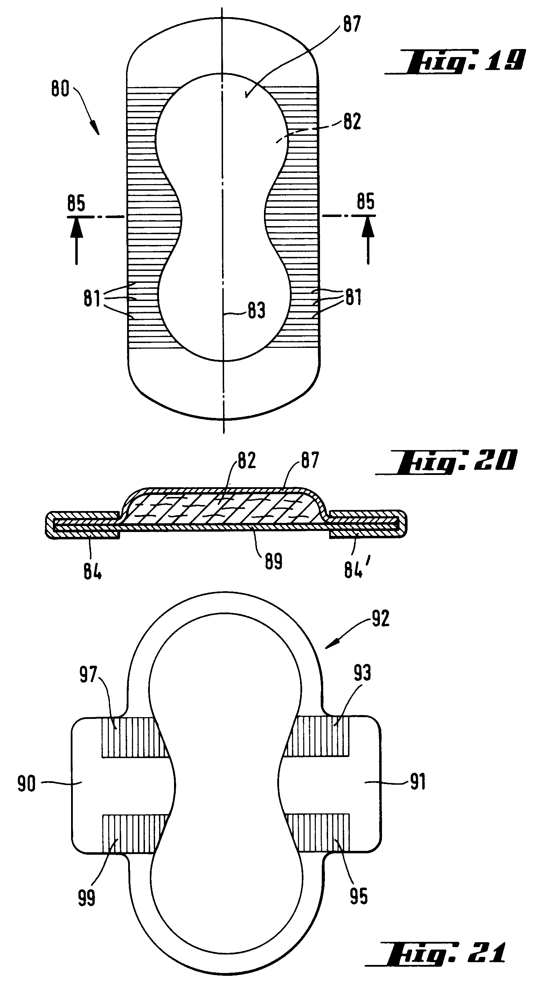

Figure 19 shows an embodiment of a sanitary napkin 80 having a shaped core 82, which is encased between a straight-sided topsheet 87 and backsheet 89. Along the longitudinal edges of the sanitary napkin,two longitudinal composite elastic members 84, 84' are C-folded around the edges of the napkin and adhesively attached thereto. This is shown in Figure 20. Physical deformations are imparted to the composite elastic members 84,84' along a number of parallel lines 81 extending perpendicular to the longitudinal center line of the sanitary napkin 80. This imparts longitudinal elasticity to the areas that are covered by the composite elastic members 84,84'.

Figure 21 shows a sanitary napkin 92 which has a composite elastic member attached to its garment-facing surface, the composite elastic member being generally unextensible outside areas 93,95,97 and 99. In the areas 93-99, the composite elastic member has been provided with extensibility, for instance by the process as shown in figure 9. The composite elastic member can direcly after having been provided with extensible areas 93-99 be connected to the sanitary napkin 92 or can be first stored on a supply roll 35 and subsequently be shipped to a manufacturing location.

Preferably, the composite elastic member as shown in figure 21, is a separate layer, directly attached to the backsheet of the sanitary napkin 92.

The elastically extensible areas 93-97 provide a good fit of the wings 90,91 around the edges of the panty and help the wings to stay in place during use.

## Claims

1. Method of making an absorbent article, the article having a liquid-pervious topsheet, a liquid-impervious backsheet and an absorbent core placed therebetween, the method comprising the steps of:
applying an elastication means to the topsheet, the backsheet, the core or any combination thereof, the elastication means comprising a composite member having a first, relatively unextensible layer and a second, elastically extensible layer, characterized by
- supplying the composite member from a supply station,
- transporting the composite member from the supply station to the topsheet, the backsheet, the core or any combination thereof in a direction of transport, the second, elastically extensible layer of the composite member being during transport attached to the first layer in a relatively unextended state, the composite member being during transport relatively unelongatable at least in the direction of transport,
followed by imparting a physical deformation to the composite member to render the composite member elastically elongatable.

2. Method of making an absorbent article, the article having a liquid-pervious topsheet, a liquid-impervious backsheet and an absorbent core placed therebetween, the method comprising the steps of:
applying an elastication means to the topsheet, the backsheet, the core or any combination thereof, the elastication means comprising a composite member having a first, relatively unextensible layer and a second, elastically extensible layer, characterized by
- supplying the composite member from a supply station,
- transporting the composite member from the supply station to the topsheet, the backsheet, the core or any combination thereof in a direction of transport, the composite member being during transport relatively unelongatable in the direction of transport, followed by
- applying the composite member to the topsheet, the backsheet, the core or any combination thereof,
wherein a physical deformation has been imparted to the composite member prior to applying it to the topsheet, the backsheet the core or any combination thereof to render at least one area of the composite member elastically elongatable such that the composite elastic member remains relatively unelongatable in the direction of transport.

3. Method according to claim 1 or 2, characterized in first applying the composite member to the topsheet, the backsheet, the core or any combination thereof, followed by imparting a physical deformation to the composite member to render the composite member elastically elongatable.

4. Method according to claim 3, wherein prior to application of the composite member the topsheet, the backsheet, the core or any combination thereof have been physically deformed to impart extensibility thereto.

5. Method according to claim 3 wherein a physical deformation is imparted simultaneously to the the composite member and to the topsheet, the backsheet, the core or any combination thereof.

6. Method according to claim 1 or 2, characterized in first imparting a physical deformation to the composite member to render the composite member elastically elongatable in the direction of transport, followed by attaching the composite member to the topsheet, the backsheet, the core or any combination thereof.

7. Method according to claim 6, characterized in attaching the composite member in a pre-stretched state to the topsheet, the backsheet, the core or any combination thereof.

8. Method according to claim 6, characterized in gathering the the topsheet, the backsheet, the core or any combination thereof, before attaching the composite member thereto.

9. Method according to any of the previous claims, characterized in that the method of imparting a physical deformation to the composite member comprises applying a pull force in the longitudinal direction of the composite member.

10. Method according to any of claims 1 to 9, characterized in that the method of imparting a physical deformation to the composite member comprises contacting the composite member with corrugated deformation means.

11. Method according to any of the previous claims, characterized in that the physical deformation is imparted only to the first, relatively unextensible layer, the second, elastically extensible layer remaining substantially undeformed.

12. Absorbent article comprising a liquid-pervious topsheet, a liquid-impervious backsheet and an absorbent core placed therebetween, at least one elaticated zone and elatication means comprising a composite member having a first, relatively unextensible layer other than the topsheet or the backsheet, and a second, elastically extensible layer which has been attached to the first layer in a relatively unextended state, the first layer having been provided with at least a physically deformed area in which the first layer is elongatable.

13. Absorbent article according to claim 12, wherein the topsheet and the backsheet are physically deformed in the elasticated zone.

14. Composite member comprising a first, relatively unelongateble layer and a second, elastically elongatable layer, which is attached to the first layer in its unextended state, the first and second layer being relatively flat, characterized in that the composite member is stored in its relatively unelongatable state in a supply configuration for supply to a production line for the formation of an absorbent article.

15. Composite member according to claim 14, wherein the composite member is provided with a physical deformation in at least one area.

16. Compostite member according to claim 14 or 15, wherein the first layer comprises a nonwoven material.

17. Composite member according to claim 14 or 15, wherein the first layer comprises a film of thermoplastic material.

18. Composite member according to claim 14 or 15, wherein the first, relatively unextensible layer and the second, elastically extensible layer are co-extruded layers.

19. Composite member according to claim 14,15, 16 or 17, wherein the second, elastically extensible layer comprises a nonwoven material.

20. Composite member according to claim 14, 15, 16 or 17 wherein the second, elastically extensible layer comprises an adhesive coating.
